# EUROPÄISCHE PATENTSCHRIFT

(11) **EP 1 259 467 B1**
(45) Veröffentlichungstag und Bekanntmachung des Hinweises auf die Patenterteilung: **27.07.2005**
(21) Anmeldenummer: 01915062.2
(22) Anmeldetag: 28.02.2001
(51) Int. Cl.: C04B 38/00, C04B 38/10, A61L 9/04, A61L 9/16, C05G 3/00

(54) **VERFAHREN ZUR HERSTELLUNG EINES MIT FLÜCHTIGEN SUBSTANZEN BELADENEN TRÄGERMATERIALS UND VERWENDUNG DESSELBEN**
METHOD FOR PRODUCING A SUPPORT MATERIAL LOADED WITH VOLATILE SUBSTANCES AND USE THEREOF
PROCEDE DE PRODUCTION DE MATIERE SUPPORT CHARGEE DE SUBSTANCES VOLATILES ET SON SON UTILISATION

(30) Priorität: 02.03.2000 DE 10010210
(43) Veröffentlichungstag der Anmeldung: 27.11.2002
(73) Patentinhaber: Mars Inc., McLean, VA 22101 (US)
(72) Erfinder: LANGE, Dietmar, 56179 Vallendar (DE); SCHLIMM, Peter, 41812 Erkelenz (DE)
(74) Vertreter: Goddar, Heinz J., Dr.
(86) Internationale Anmeldenummer: PCT/DE2001/000758
(87) Internationale Veröffentlichungsnummer: WO 2001/064601

(56) Entgegenhaltungen:
- EP-A- 0 427 704
- EP-A- 0 758 633
- EP-A- 0 965 541
- DE-A- 2 648 119
- DE-A- 4 340 277
- US-A- 5 034 222

## Beschreibung

Die Erfindung betrifft Verfahren zur Herstellung von mit flüchtigen Substanzen beladenen porösen Trägermaterialien.

Zur Abgabe von flüchtigen Substanzen an die Umgebungsluft sind insbesondere Kunststoffschäume bekannt, die mit Dufrstoffen imprägniert sind.

Aus der DE 43 40 277 A1 ist die Verwendung eines gebrannten, eine offene Mikroporosität aufweisenden keramischen Fonnkörpers zur Aufnalzme von Flüssigkeiten und anschließender Abgabe von Flüssigkeiten durch Verdunstung bekannt.

Gegenüber diesem Stand der Technik ist es Aufgabe der vorliegenden Erfindung, ein Verfahren zur Herstellung eines umweltfreundlichen Trägers bereitzustellen, der absorbierte flüchtige Substanzen aufweist und die enthaltenen flüchtige Substanzen über einen langen Zeitraum mit annähernd gleichbleibender Rate abgeben kann, sowie dessen Herstellung.

Die erfindungsgemäße Lösung besteht darin, flüssige flüchtige Substanzen auf poröses Keramikmaterial aufzubringen, das eine geeignete Porengröße bzw. Porenstruktur aufweist, um diese Substanzen aufzunehmen. Die flüchtigen Substanzen können auch in wäßriger oder/und organischer Lösung aufgebracht werden. Als flüchtige Substanzen bieten sich insbesondere Aromastoffe, Insektenrepellenzien sowie solche flüchtigen Substanzen an, die mit störenden Geruchsstoffen reagieren können.

Die Trägermatrix ist poröses Keramikmaterial, das eine geeignete Porengröße bzw. -beschaffenheit hat, wie z.B. gemäß EP 0 427 704 B1 hergestelltes Keramikgranulat. Dieses Trägermaterial wird aus 45 bis 80 Gew.-Teilen Ton, 0 bis 50 Gew.-Teilen Kalkspat, Quarzsand, Dolomit. Feldspat, Schamotte und/oder Sepiolith, 15 bis 30 Gew.-Teilen Wasser, 0,01 bis 0,03 Gew.-Teilen Verflüssigungsmittel sowie 0,001 bis 0,01 Gew.-Teilen Tensid hergestellt, die gemischt werden und in einer Dispergieranlage bei einem Druck von 1 bis 10 bar und einer Temperatur von Raumtemperatur bis 95°C aufgeschäumt werden. Ggf. können zur Herstellung einer hochviskosen thixotropen und eigenstabilen Schaumsuspension dem Verflüssigungsmittel entgegenwirkende Mittel eingesetzt werden und daraufhin die geschäumte Mischung zu Tonformkörper geformt werden. Die Tonformkörper werden zwischen 600 und 1500°C. vorzugsweise 700 bis 800°C gebrannt. Die gleichmäßige Porenstruktur mit Porengrößen von < 1 mm wird durch Aufschäumen der Ausgangsmischung bei einem Überdruck von mindestens 0.3 bar erzielt, wobei der Druck bis zum Austrag aus der Düse aufrechterhalten wird.

Die Vorteile dieses porösen Keramikträgers hinsichtlich der Umweltverträglichkeit liegen darin, daß er verwittern kann und sogar kompostierbar ist. Bei Verwendung ist der mit flüchtigen Substanzen beladene Träger formstabil und das Trägermaterial ist selbst nicht entflammbar. Die sehr große innere Oberfläche bzw. das große innere Volumen des porösen Keramikträgers ermöglicht es, eine große Masse flüchtiger Substanzen aufzunehmen, die über die gleichmäßige Porenstruktur über einen langen Zeitraum abgegeben werden kann. Demzufolge ermöglicht es das vorliegende beladene Trägermaterial, aufgenommene flüchtige Substanzen mit gleichmäßiger Rate über einen langen Zeitraum an die Umgebungsluft abzugeben.

Überraschenderweise wurde gefunden, daß das poröse Keramikträgermaterial auch zum Aufnehmen lipophiler flüchtiger Substanzen geeignet ist, obwohl das Keramikmaterial selbst keine organische Polymeroberfläche mit lipophilen Eigenschaften aufweist, sondern aufgrund seiner Zusammensetzung stark polar ist. Ohne als Einschränkung daran gebunden zu sein, wird vermutet, daß allein die günstige Porenstruktur des Keramikmateriales die Aufnahme einer sehr großen Menge flüchtiger Substanzen und deren gleichmäßige lang anhaltende Abgabe an die Umgebungsluft ermöglicht.

Das Aufbringen von bzw. Imprägnieren mit flüchtigen Substanzen kann durch Aufsprühen der flüchtigen Substanzen oder deren Lösungen auf das poröse Trägermaterial erfolgen. Als Lösungsmittel bieten sich abhängig von den verwendeten flüchtigen Substanzen Wasser und/oder organische Lösungsmittel an. Nach Aufsprühen der flüchtigen Substanzen bzw. deren Lösungen auf den keramischen Träger kann das Lösungsmittel durch Abdampfen bei Raumtemperatur oder erhöhter Temperatur entfernt werden. Ein solches Besprühen des Keramikträgers kann wiederholt werden, um eine höhere Beladung des Trägermateriales zu erzielen.

Ein besonders bevorzugtes Verfahren zum Aufbringen flüchtiger Substanzen oder deren Lösungen auf das Trägermaterial besteht darin, daß Trägermaterial bei reduziertem Druck, z.B. 200 mbar, bevorzugter 40 mbar zu besprühen, eine gewisse Haltezeit von beispielsweise 7, bevorzugt 5, besonders bevorzugt 2 Minuten bei dem reduzierten Druck anzuwenden und anschließend das besprühte Trägermaterial auf Umgebungsdruck zu belüften. Der Druck kann vor, während oder nach Aufbringen der flüchtigen Substanzen oder deren Lösung auf das Trägermaterial reduziert werden. Lösungsmittel, das zum Aufbringen der flüchtigen Substanzen verwendet wurde, läßt sich vorteilhafterweise noch unter reduziertem Druck abdampfen, um allein die gewünschten Substanzen im Trägermaterial zurückzubehalten. Auch bei Verwenden eines reduzierten Drukkes zum Aufbringen flüchtiger Substanzen auf das Trägermaterial kann nach dem Belüften auf Umgebungsdruck eventuell verwendetes Lösungsmittel abgedampft werden, wobei ggf. eine Erwärmung über Umgebungstemperatur hinaus vorgesehen werden kann. Auch das Aufbringen flüchtiger Substanzen mit Verwenden reduzierten Druckes läßt sich mehrfach hintereinander einsetzen, um eine höhere Beladung des Keramikträgers mit der flüchtigen Substanz zu erzielen. Insbesondere vorteilhaft ist die mehrfache Behandlung des porösen Trägers, wenn die flüchtigen Substanzen in Lösung aufgebracht werden.

Die erfindungsgemäßen mit flüchtigen Trägersubstanzen beladenen Trägermaterialien können sowohl als Raumluftverbesserer verwendet werden, als auch zur Verbesserung, vorzugsweise Vermeidung, von schlechten Gerüchen, die von Abfallsammelstellen, wie beispielsweise Mülleimern oder Sammelbehältern für biologische Abfälle und für Verpackungen, die zur Wiederaufarbeitung geeignet sind, abgegeben werden.

Die vorliegende Erfindung soll nun anhand von Beispielen näher erläutert werden:

### Beispiel 1: Duftzusammensetzung zur Anwendung als Raumluftverbesserer, die auf poröses Trägermaterial aufgebracht wird:

| | |
|---|---|
| Elektrolytische Zusammensetzung von natürlichen und naturidentischen duftenden Ölen, davon 35 % - 65 % natürliche Öle und 35 % - 65 % naturidentische Öle | Etwa 20 Gew.-% |
| Tenside auf natürlicher Rohstoffbasis oder natürliche Tenside | Etwa 10 Gew.-% |
| Wasser | Auf 100 Gew.% |

Die Zusammensetzung der verwendeten duftenden Öle läßt sich in Abhängigkeit von der erwünschten Duftrichtung einstellen. Insbesondere sind hier natürlich vorkommende oder naturidentische Duftstoffe geeignet, wie insbesondere Blumendüfte, frisch duftende Aldehyde, fruchtig duftende Ester etc., wie dem Fachmann auf dem Gebiet der Düfte bekannt ist.

### Beispiel 2: Poröses Trägermaterial als Trägermatrix:

Gemäß EP 0 427 704 hergestelltes Keramikgranulat aus 45 - 80 Gew.-Teilen Ton, bis 50 Gew.-Teilen Kalkspat, Quarzsand, Dolomit, Feldspat, Schamotte, Sepiolit als Zuschlagstoff, 15 - 30 Gew.-Teilen Wasser, 0,01 bis 0,03 Gew.-Teilen Verflüssigungsmittel sowie 0,001 bis 0,1 Gew.-Teilen Tensid, die gemischt werden, und in einer Dispergieranlage bei einem Druck von 1 bis 10 bar und einer Temperatur von Raumtemperatur bis 95°C aufgeschäumt ist und als Tonformkörper zwischen 700 bis 800°C gebrannt (wie zuvor beschrieben), wird bei 200 mbar mit Duftzusammensetzung besprüht, für 5 Minuten bei diesem reduzierten Druck gehalten und anschließend auf Umgebungsdruck belüftet.

### Beispiel 3: Verwendung porösen Trägermaterials, das mit einer Duftzusammensetzung nach Beispiel 1 beladen wurde:

Eine Duftzusammensetzung nach Beispiel 1 wird zu 10 bis 30 Gew.-%, bevorzugt 20 - 30 Gew.-%, besonders bevorzugt 22 - 25 Gew.-% in die Trägermatrix eingebracht. Überraschenderweise werden bei dieser Verwendung die duftenden Bestandteile vollständig von einer Pflanze aufgenommen, wenn deren Wurzeln zumindest teilweise mit dem beladenen Träger in Kontakt stehen, ohne daß jedoch die Wurzeln geschädigt werden.

### Beispiel 4: Verwendung von porösen Trägermaterialien, die mit einer Duftzusammensetzung beladen sind, zur Vermeidung von unangenehmen Gerüchen bei Sammeltonnen für biologische Abfälle (sogenannte Biotonnen):

Bei 70 Sammeltonnen mit 200 1 Volumen für biologische Abfälle wurden jeweils etwa 40 ml des erfindungsgemäßen porösen Trägermaterials, das gemäß Beispiel 2 mit einer Duftzusammensetzung von etwa 8 - 10 Gew.-% beladen war, an der Deckelinnenseite festgelegt. Um den Durchtritt bzw. das Verdampfen von Duftbestandteilen zu ermöglichen, befand sich das beladene Trägermaterial in einem Siebkorb, der an der Innenseite des Deckels der Sammeltonne befestigt war. Zur Unterbringung des erfindungsgemäßen, mit Duftzusammensetzung beladenen porösen Trägermaterials eignen sich auch Siebbeutel oder Säckchen aus durchlässigem Gewebe sowie porösen Kunststoffen.

Die Geruchsabgabe nach zwei Wochen war bei 89 % der Sammeltonnen für biologische Abfälle gemäß der Beurteilung der Verwender in ausreichendem Maße herabgesetzt, um unterhalb einer störenden Geruchsabgabe zu bleiben.

Die in der vorangehenden Beschreibung, in der Zeichnung sowie in den Ansprüchen offenbarten Merkmale der Erfindung können sowohl einzeln als auch in beliebigen Kombinationen für die Verwirklichung der Erfindung in ihren verschiedenen Ausführungsformen wesentlich sein.

## Patentansprüche

1. Verfahren zur Herstellung eines mit flüchtigen Substanzen beladenen porösen Keramikgranulats, bei dem eine Mischung von
45 bis 80 Gew.-Teilen Ton,
0 bis 50 Gew.-Teilen Kalkspat, Quarzsand, Dolomit, Feldspat, Schamotte und/oder Sepiolith
15 bis 30 Gew.-Teilen Wasser,
0,01 bis 0,03 Gew.-Teilen Verflüssigungsmittel und
0,001 bis 0,01 Gew.-Teilen
Tensid
in einer Dispergieranlage bei 1 bis 10 bar Druck und einer Temperatur von Raumtemperatur bis 95°C bei einem Überdruck von mindestens 0,3 bar, der bis zum Austrag aus der Düse aufrechterhalten wird, aufgeschäumt wird, gegebenenfalls zur Herstellung einer hoch viskosen, thixotropen und eigenstabilen Schaumsuspension dem Verflüssigungsmittel entgegenwirkende Mittel eingesetzt werden, daraufhin die geschäumte Mischung zu Tonformkörpern geformt wird, diese bei 600 bis 1500°C gebrannt werden und anschließend mindestens eine flüssige flüchtige Substanz in einer Zusammensetzung auf das Keramikgranulat aufgebracht wird, die 10 bis 90 Gew.-% flüchtige Substanz, bis zu 15 Gew.-% Tensid und bis 70 Gew.-% Wasser enthält.

2. Verfahren nach Anspruch 1, **dadurch gekennzeichnet, daß** die flüchtige Substanz bei Umgebungsdruck aufgebracht wird.

3. Verfahren nach Anspruch 1, **dadurch gekennzeichnet, daß** die flüchtige Substanz bei einem reduzierten Druck von 200 mbar aufgebracht wird.

4. Verfahren nach Anspruch 3, **dadurch gekennzeichnet, daß** die flüchtige Substanz bei einem reduzierten Druck von 40 mbar aufgebracht wird.

5. Verfahren nach einem der Ansprüche 1 bis 4, **dadurch gekennzeichnet, daß** mindestens eine flüchtige lipophile Substanz auf das Granulat aufgebracht wird.

6. Verwendung von Trägermaterial, das nach einem Verfahren der vorangehenden Ansprüche hergestellt wurde, als Raumluftverbesserer.

7. Verwendung von Trägermaterial, das nach einem Verfahren der vorangehenden Ansprüche hergestellt wurde, zur Verbesserung des Geruchs, der von Sammelbehältern für Müll oder biologische Abfälle ausgeht.

8. Verwendung von Trägermaterial, das nach einem Verfahren der vorangehenden Ansprüche hergestellt wurde, als Zusatz zur Pflanzenerde.

## Claims

1. A method of producing a porous ceramic granulate loaded with volatile substances, in which a mixture of
45 to 80 parts by weight clay,
0 to 50 parts by weight calcite, quartz sand, dolomite, feldspar, chamotte and/or spiolite,
15 to 30 parts by weight water,
0.01 to 0.03 parts by weight de-flocculating agent and
0.001 to 0.01 parts by weight surfactant
in a dispersing apparatus at a pressure of 1 to 10 bar and a temperature from room temperature up to 95 ° C is foamed at an excess pressure of at least 0.03 bar which is maintained until discharge from the nozzle, means counteracting the de-flocculating agent being optionally added, to produce a highly viscous, thixotropic and inherently stable foam suspension, the foamed mixture is then formed into moulded clay bodies, these are fired at 600 to 1500°C and a volatile liquid substance is then applied to the ceramic granulate in a composition which contains 10 to 90 % by weight of volatile substance, up to 15% by weight surfactant and up to 70% by weight water.

2. A method according to claim 1, **characterized in that** the volatile substance is applied at ambient pressure.

3. A method according to claim 1, **characterized in that** the volatile substance is applied at a reduced pressure of 200 mbar.

4. A method according to claim 3, **characterized in that** the volatile substance is applied at a reduced pressure of 40 mbar.

5. A method according to any of claims 1 to 4, **characterized in that** at least one volatile lipophilic substance is applied to the granulate.

6. Use of a support material which has been produced according to a method of the preceding claims as an air freshener.

7. Use of a support material which has been produced according to a method of the preceding claims for improving the odour which arises from collecting containers for refuse or biological waste.

8. Use of a support material which has been produced according to a method of the preceding claims as an additive to compost.

## Revendications

1. Procédé de production d'un granulat de céramique poreux chargé de substances volatiles, dans lequel
- on fait mousser un mélange constitué de :
- 45 à 80 parties en poids d'argile,
- 0 à 50 parties en poids de spath calcaire, sable quartzeux, dolomite, feldspath, chamotte et/ou sépiolite,
- 15 à 30 parties en poids d'eau,
- 0,01 à 0, 03 partie en poids d'agent fluidifiant, et
- 0,001 à 0,01 partie en poids d'agent tensioactif,
dans une installation de dispersion sous une pression de 1 à 10 bars et à une température allant de la température ambiante à 95 °C, avec une surpression d'au moins 0,3 bar, qui est maintenue jusqu'à la sortie de la buse ;
- on utilise, le cas échéant, pour la production d'une mousse très visqueuse, thixotrope et stable par elle-même, un agent agissant dans un sens opposé au sens dans lequel agit agent fluidifiant,
- on forme ensuite le mélange expansé en mousse en corps moulés en argile, on cuit ceux-ci à 600 à 1500 °C puis on applique ensuite sur le granulat de céramique au moins une substance volatile liquide sous forme d'une composition qui contient 10 à 90 % en poids de substance volatile, jusqu'à 15 % en poids de tensioactif et jusqu'à 70 % en poids d'eau.

2. Procédé selon la revendication 1, **caractérisé en ce que** la substance volatile est appliquée à la pression ambiante.

3. Procédé selon la revendication 1, **caractérisé en ce que** la substance volatile est appliquée sous une pression réduite de 200 mbars.

4. Procédé selon la revendication 3, **caractérisé en ce que** la substance active est appliquée sous une pression réduite de 40 mbars.

5. Procédé selon l'une quelconque des revendications 1 à 4, **caractérisé en ce qu'**on applique au moins une substance lipophile volatile sur le granulat.

6. Utilisation de matière support, qui a été fabriquée selon un procédé des revendications précédentes, comme améliorateur d'air ambiant.

7. Utilisation de la matière support qui a été fabriquée selon un procédé des revendications précédentes, pour améliorer l'odeur qui se dégage des conteneurs de collecte d'ordures ménagères ou de déchets biologiques.

8. Utilisation de la matière support, qui a été produite selon un procédé des revendications précédentes, comme additif à la terre végétale.
